# EUROPEAN PATENT APPLICATION

(11) **EP 4 659 653 A1**
(43) Date of publication of application: **10.12.2025**
(21) Application number: 24305891.4
(22) Date of filing: 06.06.2024
(51) Int. Cl.: A61B 3/06, A61B 5/37

(54) **METHOD FOR OBJECTIVELY ASSESSING DISCOMFORT GLARE OR DISABILITY GLARE OF A SUBJECT AND CORRESPONDING DEVICE AND EYEWEAR**

(71) Applicant: Essilor International, 94220 Charenton-Le-Pont (FR)
(72) Inventor: TARTAGLIA, Elisa, 75011 PARIS (FR); SALVATI, Valerio, 75012 PARIS (FR)
(74) Representative: Jacobacci Coralis Harle

(57) **Abstract**

Method for objectively assessing discomfort glare or disability glare of a subject, comprising the steps of (a) measuring and recording a cortical activity signal of the subject while at least one eye of the subject receives a given light condition during a time comprised between 100 ms and 500 ms, (b) processing the recorded cortical activity signal to obtain a useful signal and to identify, from the useful signal, at least one specific cortical signature, c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state. The useful signal which is used to identify the specific cortical signature in step (b) comprises a gamma frequency band between 30 Hz and 49 Hz.

## Description

### TECHNICAL FIELD OF THE INVENTION

The domain of the invention delves into the realm of light sensitivity. The invention relates to a method for objectively assessing discomfort glare or disability glare of a subject and to a corresponding device. It has applications in the fields of ophthalmology and optometry.

### BACKGROUND INFORMATION AND PRIOR ART

The physiological and neurological responses of human beings to light exposure, notably to various light conditions including intensities, have been explored and is now better understood.

This understanding is critical in that the spectrum of light sensitivity can range from mild discomfort in bright conditions, often manifested as a mere squint, to severe pain or headaches triggered by even minimal light exposure.

Furthermore, the implications of this field are vast. Beyond the immediate physiological responses, understanding light sensitivity can lead to innovations in lighting design, advancements in therapeutic interventions for conditions like migraines, and the development of protective eyewear tailored to individual needs.

The determination of the glare state is generally done with experiments for assessing the discomfort or disability glare and in which a human subject, which is subjected to different light conditions, has to answer whether he/she feels a discomfort glare and the level of the discomfort.

Current metrics and measurements for evaluating discomfort glare are mainly derived from subjective evaluations, using scales such as Hopkinson's or De Boer's, with De Boer's 9-level scale, ranging from negligible (level 9) to unbearable (level 1), being the most commonly used in the literature.

This is therefore a highly subjective experimenting and measurement.

There is no standardized and objective test for evaluating discomfort glare.

So far, solutions have been proposed to solve this problem but with limited success.

In those solutions, measures of the physiological responses induced by the light are analyzed in order to try to objectify evaluate the perceived glare. These measures include pupil size, eye movements, gaze direction, eye opening degree, and blink frequency.

It has been shown that each of these solutions has limitations, and more objective solutions still need to be proposed to generate more robust metrics.

In addition, in those solutions, no physiological signature of the effects of optical filters on glare has been identified yet.

Document WO2022058456A1 discloses a method for assessing objectively a glare state of a subject.

### SUMMARY OF THE INVENTION

The goal of the current invention is to provide an objective and more efficient solution thanks to a reliable and unambiguous signature of glare and reliable assessment of glare from recordings of the cortical activity of the subject.

Thanks to the proposed objective solution of assessing glare, it is possible to offer to each user the most appropriate ophthalmic solution, such as filters.

A first object of the invention is to provide a method for objectively assessing discomfort glare or disability glare of a subject, comprising the steps of:
a) measuring and recording a cortical activity signal of the subject while at least one eye of the subject receives a given light condition during a time comprised between 100 ms and 500 ms,
b) processing the recorded cortical activity signal to obtain a useful signal and to identify, from the useful signal, a specific cortical signature indicative to glare states,
c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state.

According to the invention, the useful signal which is used to identify the specific cortical signature in step b) comprises a gamma frequency band between 30 Hz and 49 Hz.

As a consequence, the duration of the measuring and recording and of the receiving of the given light condition is comprised between 100 ms and 500 ms.

In various embodiments of the method, the following means and characteristics, which can be used alone or in any technically possible combination, are used:
- the disability glare state corresponds to the case the subject has to close his/her eye(s),
- the discomfort glare state corresponds to the case the subject is not comfortable with the light but can keep his/her eye(s) open,
- the no-glare (i.e. non-glare) state corresponds to the case the subject is comfortable with the light,
- preferably, the duration of the measuring and recording and of the receiving of the given light condition is 500 ms,
- the method is limited to assessing discomfort glare of a subject,
- the method is limited to assessing disability glare of a subject,
- the condition of the given light condition is an intensity of the light,
- the intensity of the light for the given light condition is subject dependent,
- the intensity of the light that is subject dependent is an intensity for which the subject is in a no-glare state,
- the intensity of the light that is subject dependent is an intensity for which the subject is in a discomfort glare state,
- the intensity of the light that is subject dependent is an intensity for which the subject is in a disability glare state,
- the intensity of the light for the given light condition is 45 lux,
- the measuring and recording of the cortical activity signal start from the moment the subject starts receiving the given light condition,
- the measuring and recording of the cortical activity signal stops at the moment the given light condition ceases,
- the useful signal which is used to identify the specific cortical signature in step b) is limited to the gamma frequency band between 30 and 49 Hz to the exclusion of any other frequencies,
- the processing of the recorded cortical activity signal is a filtering,
- the useful signal is a filtered recorded cortical activity signal,
- the filtered recorded cortical activity signal is filtered with a high-pass filter at 30 Hz and a low-pass filter at 49 Hz,
- the filtered recorded cortical activity signal is filtered with a band-pass filter from 30 Hz to 49 Hz,
- the processing of the recorded cortical activity signal is a filling-in,
- the recorded cortical activity signal is processed to detect, remove and replace artifacts with filling in through interpolation,
- the processing of the recorded cortical activity signal is an averaging with iteratively measured and recorded cortical activity signals and/or with iteratively obtained useful signals,
- in the iterations, the given light condition remains the same,
- in the iterations, the measuring and recording time remains the same,
- in the iterations, both the given light condition and the measuring and recording time remain the same,
- the useful signal for the identification is an average of a number of iteratively recorded cortical activity signals measured and recorded at step a) or of obtained useful signals at step c),
- the useful signal for the identification is an average of a minimum of five to twenty-five iteratively recorded cortical activity signals or obtained useful signals,
- the useful signal for the identification is an average of a minimum of five iteratively recorded cortical activity signals or obtained useful signals,
- advantageously, the useful signal is an average of all iteratively recorded cortical activity signals or obtained useful signals,
- the cortical activity signal is measured by an Electroencephalographic system (EEG),
- the cortical activity signal measured by an Electroencephalographic system (EEG), is measured and recorded as channels,
- the measured and recorded channel(s) is/are at least one channel from the frontal and/or occipital channels,
- the at least one channel from the frontal and/or occipital channels is selected from FPz, FP1, FP2, O1, Oz channels,
- the at least one channel from the frontal and/or occipital channels is selected from FPz, FP1, FP2, Fz, O2 channels,
- the measured and recorded channels are frontal and occipital channels,
- the measured and recorded frontal and occipital channels are FPz, FP1, FP2, O1, Oz,
- the measured and recorded frontal and occipital channels are : FPz, FP1, FP2, Fz, 02,
- at step a), the at least one eye of the subject is provided with a filter through which the light passes before reaching the at least one eye of the subject,
- the useful signal is recorded as sampled digital values,
- at step b), the useful signal is processed to identify at least one specific cortical signature and at step c), the determination is performed by comparing each of the identified specific cortical signature(s) to at least one corresponding predefined threshold parameter,
- at step b), the useful signal is processed to identify a plurality of specific cortical signatures and at step c), the determination is performed by comparing each of the identified specific cortical signatures to a corresponding predefined threshold parameter,
- steps b) and c) are executed with a machine learning process,
- the cortical activity signal which is measured and recorded in step a) comprises regional parts originating from a plurality of different regions of the cortex and wherein the useful signal which is used to identify the specific cortical signature in step b) is obtained from at least these regional parts of the cortical activity signal originating from different regions of the cortex,
- the cortical activity signal which is measured and recorded in step a) comprises two regional parts originating respectively from the occipital and frontal regions of the cortex and wherein the useful signal which is used to identify the specific cortical signature in step b) is obtained mainly from these two regional parts of the cortical activity signal,
- the cortical activity signal which is measured and recorded in step a) comprises two regional parts originating respectively from the occipital and frontal regions of the cortex and wherein the useful signal which is used to identify the specific cortical signature in step b) is obtained mainly from these two regional parts of the cortical activity signal to the exclusion of any other part of the cortical activity signal which may originate from another region of the cortex,
- the cortical activity signal is measured in step a) with electrodes respectively facing the different regions of the cortex,
- the cortical activity signal which is measured and recorded in step a) comprises an occipital part originating from an occipital region of the cortex and wherein the useful signal which is used to identify the specific cortical signature in step b) is obtained mainly from this occipital part of the cortical activity signal,
- the cortical activity signal is measured in step a) with an electrode facing said occipital region of the cortex,
- the cortical activity signal which is measured and recorded in step a) comprises a frontal part originating from a frontal region of the cortex and wherein the useful signal which is used to identify the specific cortical signature in step b) is obtained mainly from this frontal part of the cortical activity signal,
- the cortical activity signal is measured in step a) with an electrode facing said frontal region of the cortex,
- the recorded cortical activity signal used for said step c) determination comprises signal recorded between 0 ms and 500 ms counted from the moment the subject start receiving said given light condition, i.e. 500 ms of signal length and signal starting when the light starts,
- the identification at step b) comprises a Fourier transformation of the useful signal,
- the specific cortical signature is a Fourier transform of the useful signal, said Fourier transform comprising frequency peaks of determined energies,
- the predefined threshold parameter is a set of values descriptive of a peak energies repartition over frequencies,
- the predefined threshold parameter of the specific cortical signature is a criterion k which is the difference of two energy values of two maximally discriminative peaks between no-glare state and discomfort glare state or disability glare state,
- the method comprises a step of recording at least one custom parameter of the subject from at least: the age of the subject, the sex of the subject, pathology(ies) of the subject, and said custom parameter is used for processing the recorded cortical activity signal at step b) and/or for selecting said threshold parameter of the specific cortical signature used at step c),
- the threshold parameter of the specific cortical signature is predetermined in a preliminary threshold determination step comprising a statistical computation of data related to cortical activities under different light conditions of a plurality of individuals from a population distinct from the subject whose discomfort or disability glare is to be assessed, in relation to the feeling of each individual as to whether he/she feels a no-glare or discomfort glare or disability glare state,
- the statistical computation of the threshold parameter is a computation of an average from the threshold parameters of the individuals,
- the specific cortical signature is predetermined in the preliminary threshold determination step,
- in the preliminary threshold determination step comprises the sub-steps of:
   -- for each individual of the population, measuring and recording a plurality of cortical activity signals of the individual while at least one eye of the individual receives respective light conditions during a time comprised between 100 ms and 500 ms,
   -- for each individual of the population and for each of the light conditions, recording the feeling of the individual as to whether he/she feels a no-glare or discomfort glare or disability glare state,
   -- processing the recorded cortical activity signals to obtain useful signals,
   -- comparing with each other the useful signals and feelings of each individual or of all the individuals of the population to statistical compute said predefined threshold parameter of the specific cortical signature for which a change occurs between no-glare, discomfort glare or disability glare states in the population of the individuals,
- the threshold parameter of the specific cortical signature is predetermined in a preliminary threshold determination step comprising a statistical computation of data related to cortical activities under different light conditions of the subject whose discomfort or disability glare is to be assessed, in relation to the feeling of the subject as to whether he/she feels a no-glare or discomfort glare or disability glare state,
- the preliminary threshold determination step comprises the sub-steps of:
   -- measuring and recording a plurality of cortical activity signals of said subject while at least one eye of the subject receives respective light conditions during a time comprised between 100 ms and 500 ms,
   -- for each of the light conditions, recording the feeling of the subject as to whether he/she feels a no-glare or discomfort glare or disability glare state,
   -- processing the recorded cortical activity signals to obtain useful signals,
   -- comparing with each other the recorded cortical activity signals and feelings of said subject to determine said predefined threshold parameter of the specific cortical signature as the recorded cortical activity signal for which a change occurs between no-glare, discomfort glare or disability glare states,
- the threshold parameter of the specific cortical signature is predetermined in a preliminary threshold determination step and in said preliminary threshold determination step the at least one custom parameter of the subject or of each of individuals is recorded and threshold parameters are predetermined in relation to the at least one custom parameter, said at least one custom parameter of the subject being from at least: the age of the subject or individual, the sex of the subject or individual, pathology(ies) of the subject or individual, in order to be able to select said threshold parameter of the specific cortical signature used at step c) according to the at least one custom parameter of the subject in the assessment of the discomfort glare or disability glare of the subject,
- the method uses at least a discomfort threshold parameter defining the shift from the no-glare state to discomfort glare state,
- the method uses at least a pain threshold parameter defining the shift from discomfort glare state to painful glare state,
- the method uses at least a disability threshold parameter defining the shift from the no-glare state to disability glare,
- the method further comprises a step of determining, based on the result the determining of step c), a parameter that is characteristic of a light filter to be provided to the subject in order to maintain or improve a visual comfort and/or a visual acuity of the subject.

A second object of the invention is to provide a device for objectively assessing discomfort glare or disability glare of a subject, comprising a control unit configured to execute the steps of:
a) measuring and recording a cortical activity signal of the subject while at least one eye of the subject receives a given light condition during a time comprised between 100 ms and 500 ms,
b) processing the recorded cortical activity signal in real time to obtain a useful signal and to identify, from the useful signal, at least one specific cortical signature,
c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state, and
in which the useful signal which is used to identify the specific cortical signature in step b) comprises a gamma frequency band between 30 Hz and 49 Hz.

In various embodiments of the device, in addition to the material means and characteristics described in the current application, the following means and characteristics, which can be used alone or in any technically possible combination, are used:
- the device is an eyewear,
- the device comprises an eyewear and at least one light source that is operated by the control unit to expose the at least one eye of the subject to said given light condition,
- the useful signal which is used to identify the specific cortical signature in step b) is limited to the gamma frequency band between 30 and 49 Hz to the exclusion of any other frequencies,
- the preliminary threshold determination step is done with a system distinct of the device,
- the preliminary threshold determination step is done with the device,
- the device further comprises at least one light source that is operated by the control unit to expose the at least one eye of the subject to said given light condition.

A third object of the invention is to provide an eyewear comprising:
- a device for objectively assessing discomfort glare or disability glare of a subject and comprising a control unit configured to execute the steps of:
   a) measuring and recording a cortical activity signal of the subject while at least one eye of the subject receives a given light condition during a time comprised between 100 ms and 500 ms,
   b) processing the recorded cortical activity signal to obtain a useful signal and to identify, from the useful signal, at least one specific cortical signature,
   c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state, and
   in which the useful signal which is used to identify the specific cortical signature in step b) comprises a gamma frequency band between 30 Hz and 49 Hz, and
- an active filter having a variable range of transmission,
wherein the control unit is further adapted to determine a value of said variable range of transmission of the active filter based on the result of the assessment by said device of the discomfort glare or disability glare state.

Advantageously for the eyewear, the useful signal which is used to identify the specific cortical signature in step b) is limited to the gamma frequency band between 30 and 49 Hz to the exclusion of any other frequencies.

### List of figures

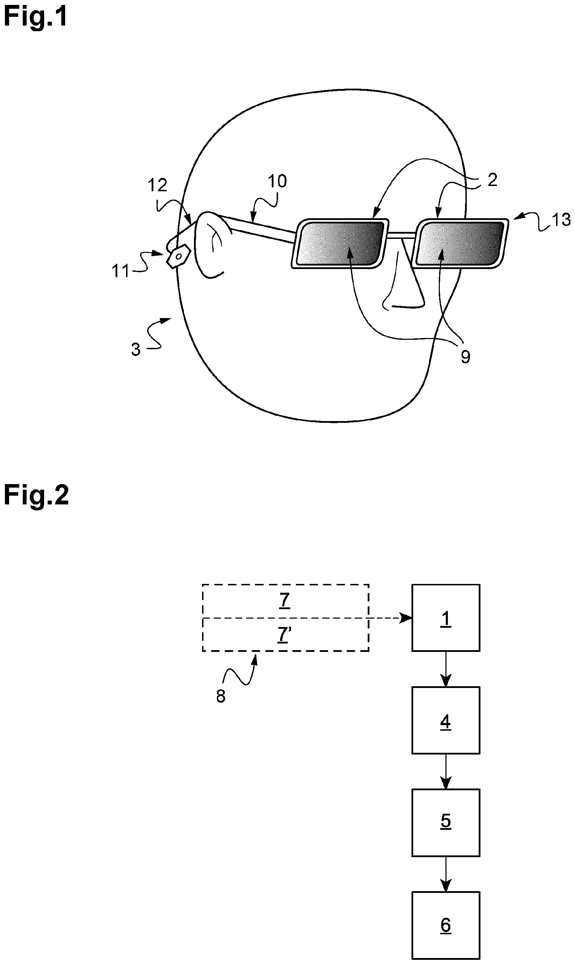

Figure 1 is a schematic view of an embodiment of a device designed to objectively assess in-real-time a subject's response to light regarding the glare and that features lenses that can dynamically change colors or transmission index or employ filters and also features at least one electrode to measure a cortical activity signal of the subject wearing the device,
Figure 2 is a flowchart of the method of the invention,

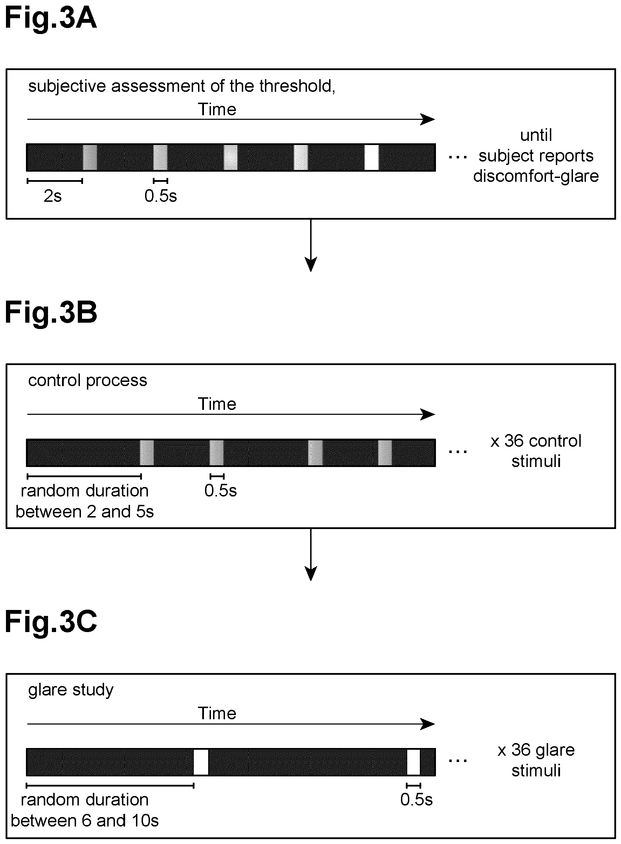

Figures 3A, 3B and 3C are schematic representations of respectively, the subjective assessment of a glare threshold, the control process (i.e. with a non-glare condition) and the glare study (i.e. with a discomfort-glare condition with and without filter) as regards the way the light conditions are supplied to the subject in the statistical study to compute the threshold parameter in a preliminary threshold determination step.

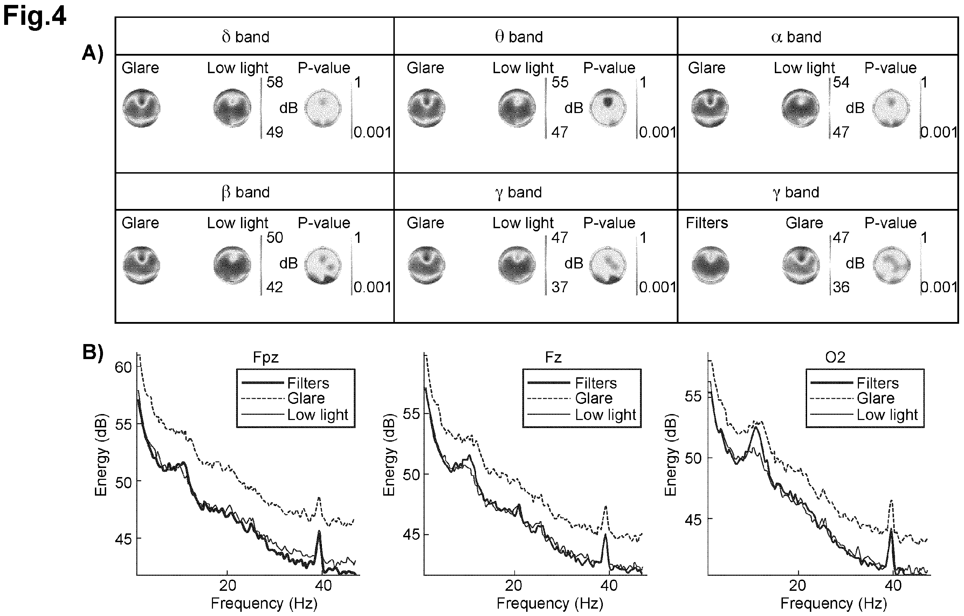

Figure 4 is an example of the results of a statistical study on individuals and considering the averaged Fourier transformed useful signals from filtered recorded cortical activity signals preferably limited to the gamma frequency band between 30 and 49 Hz.
   More precisely, the averages are done as follows: first the trials are averaged for each individual, then the Fourier transform is computed on each signal for each individual, and then the Fourier transforms are averaged together for all the individuals. In addition, as regards the frequency band, each of the measured cortical activity signal is first band-pass filtered between 1 and 49 Hz and to compute the power in each frequency band it is proceeded as follows: firstly the recorded cortical activity signal is band pass filtered again in the relevant frequency band (for example the gamma band between 30 and 49 Hz) and then the FFT is computed on that part of the band filtered signal. This is repeated for each frequency band of interest in the case of the statistical study and preferably in the band between 30 and 49 Hz in the case of the method for objectively assessing discomfort glare or disability glare.

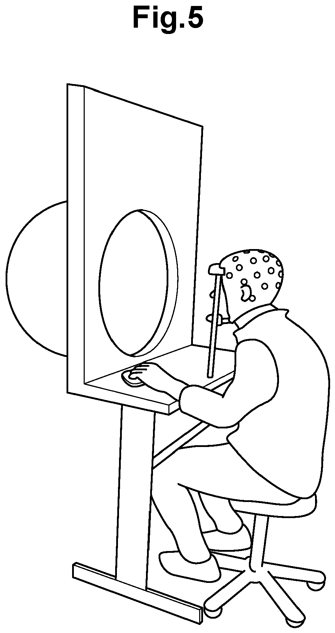

Figure 5 is a view of a device comprising a light source allowing uniform illumination of the face of an individual of subject that is represented wearing an EEG helmet.

### DETAILED DESCRIPTION OF EXAMPLE(S)

An embodiment of the device of the invention is now described in relation to figure 1. This embodiment is an eyewear 13 comprising the device for objectively assessing discomfort glare or disability glare of a subject. The device comprises a control unit configured to execute the steps required for the assessment. In figure 1 a head of a subject 3 is schematized. The subject 3 is wearing the eyewear 13 on his/her eyes 2. The eyewear 13 comprises active glasses 9, a frame 10 comprising electronic circuits and a power supply, e.g. a rechargeable battery, a connector 12. The connector 12 is connected to an electrode 11 arranged on the scalp of the subject 3, on the occipital region. The eyewear also comprise an embedded reference electrode (not visible) typically arranged on the frame to contact one of the ears of the subject 3 or contact an area of the scalp. The electronic circuits in the frame comprise the control unit. In a simpler embodiment of the eyewear, the control unit is external and the electronic circuits of the frame comprise a communication circuit to communicate with an external device comprising the control unit for objectively assessing discomfort glare or disability glare of a subject.

In this example, a single cranial electrode is placed on the back of the scalp of the head, in the occipital region, which is responsible for the processing of the visual information. This cranial electrode can be mounted on a comfortable headband worn by the subject.

In a different embodiment, three cranial electrodes can be applied to the scalp in the frontal region.

The method to determine physiologically, objectively, and unambiguously if or when a subject experiences glare is now described in relation to figure 2.

The step referenced 1 is a step of measuring and recording a cortical activity signal of the subject while at least one eye 2 of the subject 3 receives a given light condition. The step referenced 4 is a step of processing the recorded cortical activity signal to obtain a useful signal. The step referenced 5 is a step for identifying, from the useful signal, a specific cortical signature indicative to glare states and more precisely a specific cortical signature that correlates with a discomfort glare or disability glare state of the subject. Said identification comprises a Fourier transform of the useful signal, i.e. the power spectral density. The step referenced 6 is a step for determining whether the subject is or not in no-glare state or a discomfort glare or disability glare state by using the cortical signature found in the preceding step and at least one predefined threshold parameter of the specific cortical signature that is characteristic of a shift from no-glare state to discomfort glare state or disability glare state, the specific cortical signature being compared to said at least one predefined threshold parameter of the specific cortical signature. Said determination is done in the frequency domain as the specific cortical signature is a result of a Fourier transformation and the predefined threshold parameter is for example a set of values descriptive of a peak energies repartition over frequencies.

For effectiveness of the assessment, a support vector machine is preferably used.

In those steps, the signature for the specific cortical signature to be identified and the threshold parameter of the specific cortical signature are predefined data that have been determined previously on the subject itself or on a defined population of individuals thanks to a statistical study. This/those previous determinations are referenced 7 as a step for predetermining the threshold parameter of the specific cortical signature and referenced 7' as a step for predetermining the specific cortical signature in a global preliminary threshold determination step referenced 8. Note that the step 7' for predetermining the specific cortical signature in a preliminary threshold determination step 8 can be omitted if the signature is already known. Same for the step 7 for predetermining the threshold parameter of the specific cortical signature, if the threshold parameter is already known.

In the present case, the determination of the specific cortical signature does not need to be determined repeatedly as it has already been determined that it corresponds to the Fourier transformation, preferably the power spectral density, of the cortical signal.

On the contrary, the computation/determination of the threshold parameter may be done repeatedly in the long run to get results from a growing population and therefore gaining in significance level and/or done within sub-populations.

Thanks to the method and to the device of figure 1 it is also possible to determine physiologically, objectively, and unambiguously the effect of optical filters on the subject glare experience.

For that purpose the subject, without any filter on his/her eye(s), is subjected to a given light condition, i.e. intensity, which corresponds to a state of discomfort glare or disability glare as assessed from his/her measured and recorded cortical activity signal according to the method of the invention. Using said given light condition, different filters are arranged on the eye(s) of the subject and assessments according to the invention, i.e. from measured and recorded cortical activity signals, are done till a filter is found to give a result of assessment of no-glare state.

In a more advanced embodiment, notably using the eyewear 13 comprising active glasses 9 of the figure 1, the active glasses operate as filters which level of filtering can be modified by the control unit. Therefore, the assessment method can be developed into a Brain-Computer-Interface device, to pilot in real time optical lenses, e.g. E-Chromics.

Said eyewear allows the testing of filters on subjects and notably to know if a filter is reducing the glare effect. This is done by determining according to the invention whether the subject is or not in a no-glare or a discomfort glare or disability glare state with the filter in place from the power of the frequency peaks of the Fourier transformed EEG signal. For that testing of the filters, the best EEG channels (or electrodes) are : FPz, FP1, FP2, Fz, 02, which are in the frontal and occipital regions. One or more of said channels may be used for the tests of the filters.

Note that if there is no test of a filter and the method is only for objectively assessing discomfort glare or disability glare versus non-glare (i.e. to classify glare vs non-glare) of a subject without consideration to filters, the best channels (or electrodes) are : FPz, FP1, FP2, O1, Oz; which are also in the frontal and occipital regions. Again, one or more of said channels may be used for the assessment.

In both cases, tests of filters and glare assessment, this is most preferably the cortical signal frequency limited in the gamma frequency band between 30 Hz and 49 Hz that is used for the Fourier transformation.

The specific cortical signature and the threshold parameter of the specific cortical signature that are used for the assessment can be predefined on the subject or on individuals from a determined population with a statistical study.

The statistical study that allowed the determination of the specific cortical signature and the computation of the threshold parameter is now explained. Those explanation also applies to the subject itself to get at least his/her threshold parameter.

The physiological signature of glare and the effects of optical filters to relieve it found during the statistical study, is based on a plurality of signs and parameters that can be used together to provide the best signature for the identification. Those signs and parameters are notably the frequency band of the waves in the measured and recorded cortical activity signals and the time when the cortical waves have to be assessed. As regards the frequency band, it has been found that the gamma frequency analysis of brain activity is the most relevant regarding exposure to glaring light. As regards the time, it has been found that the measurement and recoding of the first 500 ms after the start of the exposure to light is the most relevant regarding exposure to glaring light.

It has also been found that the cortical waves from frontal and occipital regions of the cortex are the most relevant regarding exposure to glaring light, noting that waves from the occipital cortex region alone being sufficient.

It has also been found that the Fourier transformation of the cortical signal gives the best signature for assessing the glare. Therefore, the threshold parameter that is computed is issued from Fourier transformations characteristics and comprises values in relation to Fourier transformed signals, e.g. frequencies of peaks, energies of the peaks, repartition of the peaks over the frequency range...

For the statistical study the device represented in figure 5 can be used. It is an arrangement with a light source allowing uniform illumination of the face of the individual. On the figure an individual is sited in front of a light dome, i.e. an EFFILUXO dome, and wear an EEG helmet with electrodes for a number of channels.

In the complete statistical study a full EEG with all the channels is used to get the best specific cortical signature(s) and threshold parameter(s) and the best channel(s) (i.e. cortical area(s) ) and frequency bands. It is also possible to use a limited number of channel(s) and frequency band(s) if the best ones are already known in the case of a more specific statistical study, for example to get more precise specific cortical signature(s) and threshold parameter(s) on a greater or different population. For those last cases of specific statistical studies (which can be limited to one person, the subject itself), an eyewear 13, possibly comprising active glasses 9, such as the one of figure 1 with one electrode/channel can be used.

Finally, the device represented in figure 5 can be used for the assessment of glare on the subject itself (i.e. not for a statistical study).

The statistical study starts with a first phase of Discomfort Glare Threshold Assessment which is a subjective assessment and in which no EEG is involved. In this first phase, individuals of a defined population are exposed to varying light intensities, and their threshold level of discomfort glare (and/or disability glare according to the type of study) is recorded using a subjective scale. It's a method to determine, at a purely subjective level, the light intensity at which different individuals perceive a glare (discomfort and/or disability according to the type of study).

It has been detected a high degree of inter-individual variability in light sensitivity across the individuals of the sample population.

Then a second phase of EEG Recording Session is executed. In this second phase where for each individual of the population, the individual's cortical activity is recorded using an Electroencephalogram (EEG) across three different conditions:
- a controlled condition or low-light condition,
- a discomfort-glare condition, and
- a discomfort-glare with filter condition.

The EEG, a non-invasive technique, involves placing electrodes on the scalp of the individual to measure the cortex's electrical waves. In this particular study, the emphasis is on the frequency analysis of the recorded cortical activity.

Then a third phase of Data Cleaning is executed. In that third phase, once recorded in the second phase, the EEG data undergo a cleaning process. Indeed, the raw EEG data is often riddled with artifacts, i.e. unwanted signals that are not related to brain activity. These artifacts can arise from various sources, such as eye movements, muscle activity, or external interference. It is advantageous to remove these artifacts to ensure that the subsequent analysis is based purely on brain responses and not confounded by extraneous noise. Various techniques, both manual and algorithmic, are employed to process the data and remove these artifacts, leaving behind a clean dataset, i.e. a clean cortical signal. Preferably, said clean dataset is obtained in a similar way as the useful signal is obtained.

Then a fourth phase of Data analyzing is executed. The EEG data are analyzed to identify patterns and neural markers associated with the different levels of subjective light sensitivity. In that fourth phase, the clean dataset is analyzed in the frequency domain for each individual and his/her corresponding subjective assessment, each tested condition and each trial. This analysis in frequency implements a Fourier transformation, e.g. power spectral density, of the dataset and allows to determine the threshold parameter thanks to statistical analysis with a classifier in a fifth phase.

During the statistical study, it has been found that by looking at the power of the gamma band of the EEG one can statistically infer whether each individual has experienced discomfort-glare or is wearing a filter which relieves the discomfort-glare. The power of the gamma band is then used as feature for the classifier and the threshold parameter comprises values related to peaks of Fourier transformed signal having frequency peaks in the gamma band.

Indeed, on the upper part of figure 4 (referenced A) on the figure), it is shown the topological map of the energy for each band of the EEG signals found significant for each condition. The significant p-value areas are displayed next to each couple of conditions. All the 5 bands, i.e. alpha to gamma, are displayed next to each couple of conditions. All the 5 bands are significant for the glare condition. For the filter condition only the gamma band results are significant. For the filter condition only the gamma band is significant.

On the lower part of figure 4 (referenced B) on the figure), it is shown the power spectral density (PSD), i.e. the distribution of signal energy over the frequency spectrum, for the Low light, Glare and Filters conditions respectively for the channels FPz, Fz and 02.

As clearly visible in figure 4, the spectra of the energies of the conditions are very different. It is clear that the energy (dB unit) in the glare condition is higher than in the low-light and the filter condition. Therefore, the distribution of the energy over the frequencies is a relevant specific cortical signature and that the values of the energy are also relevant threshold parameters and that, it makes possible to take it as a feature for the machine-learning algorithm yielding high accuracies.

Therefore it can be defined a criterion k, i.e. the relevant threshold parameter(s) in this example, which is the difference of two energy values of two maximally discriminative peaks between no-glare state and a glare state, e.g. a discomfort glare state or a disability glare state, the discriminative peaks being the ones located at approximatively 40 Hz on the energy vs frequency curves of part B) of figure 4.

As regards the filter a next step is to classify the individuals trial by trial based on whether they are glared or they wear filters. The accuracy of the classification allows to establish if individual cortical activity, recorded in real time, can predict if a subject is in need to wear filters or not.

The above-mentioned phases are now described more in depth.

The first phase of Discomfort Glare Threshold Assessment consists in conducting a subjective discomfort glare threshold assessment. The subjective threshold that is recorded in that phase is an intensity value of the light. Individuals are exposed to a series of light flashes, each lasting 500 ms, with exponentially increasing intensities. These flashes were alternated with periods of darkness, lasting 2 s. Individuals signal by button press as soon as to it becomes challenging to keep their eyes open due to the light's intensity. This intensity value marks the individual-specific discomfort glare threshold. As soon as the button is pressed, the ramp stops. Individuals were asked to repeat this phase four times, to have a reliable value estimation for the subjective threshold.

It has to be noted that this subjective assessment will determine if the method of the invention is for assessing discomfort glare of a subject or for assessing disability glare of a subject or for the assessment of both as this will be reference for determining, notably in the statistical study, the specific cortical signature(s) and corresponding threshold parameter(s), those being dependent of the subjective assessment done. Therefore, it is preferable to do two subjective assessments, one for the discomfort glare and one for the disability glare (or any other level of glare that is to be objectively assessed with the method of the invention).

In the second phase of EEG recording session, several electrodes are placed in the occipital, central and frontal regions of the head to be able to measure and record cortical activity signal issued from the occipital, central and frontal regions of the cortex. Electrodes can be mounted on a comfortable headband worn by the individual. During the recording of brain activity, i.e. cortical activity signal, the individual is sitting in a dark room, in front of an illumination dome and the individual maintains fixation on the center of the illumination dome and remains as motionless as possible, with her/his head comfortably resting on a chinrest. Except when the individual is exposed to light, the individual stays in the dark/blackout, i.e. the illumination dome emits no-light.

The recording of the neural activity with EEG takes place during three distinct conditions:
- a control condition or low-light condition,
- a discomfort-glare condition, and
- a discomfort-glare with filter condition.

The control condition lasts a fixed duration of 3 min. Individuals fixate to the illumination dome's center. The individual does not perform any other action. The illumination dome projects a single light intensity of 45 lux, i.e. a non-glare intensity, for a duration of 500 ms. This is iterated and the projections of light are interleaved with dark periods of random duration, typically between 2 s and 4 s. The iterations allow to average the recorded signals. Time randomization avoids photoreceptors' adaptation and the brain's prediction mechanisms. A total of 36 trials, i.e. iterations, are recorded.

The discomfort-glare condition lasts 7 min. The illumination dome projects for a duration 500 ms a single light intensity which is the individual-specific glaring light intensity that has been recorded in the first phase of (subjective) Discomfort Glare Threshold Assessment. Again, this is iterated and the projections of light are interleaved with dark periods of random durations, typically between 6 s and 10 s.. The iterations allow to average the recorded signals. The longer durations of the dark periods for this discomfort-glare condition are chosen to maximize recovery due to the uncomfortable light intensity. The recordings of the discomfort-glare condition consist of 36 trials, i.e. iterations, divided into two sessions of 3.5 min each, with a break of 1 minute between the two.

After a break of 10 minutes, the individual is subjected to the discomfort-glare with filter condition which is similar to the preceding discomfort-glare condition but while wearing optical filters.

It can be noted that, instead of 36 trials/iterations for each of the conditions (i.e. control condition, discomfort-glare condition, discomfort-glare with filter condition), 20 trials for each condition appears to be sufficient.

Figure 3 is schematically representing the organization of the subjective assessment of the threshold (i.e. the first phase of Discomfort Glare Threshold Assessment), the control process (i.e. the control condition that is a non-glare condition) and the glare study (i.e. the discomfort-glare conditions with and without filter). The black area are the blackout periods.

In the subjective assessment of the threshold, each individual (or the subject according to the case) is subjected to light flashes, each lasting 500 ms, with exponentially increasing intensities until the individual (or subject) reports discomfort-glare. In the glare study each individual (or the subject) receives a light intensity that is individual (subject) specific and is an intensity for which he/she has reported discomfort-glare in the subjective assessment of the threshold.

In the third phase of Data Cleaning, the EEG data collected is cleaned: the initial raw recordings are filtered with a high-pass filter at 1 Hz and a low-pass filter at 49 Hz. Channels that contain artifacts are detected and filled in through interpolation.

In the fourth phase of Data analyzing, the data is processed in order to extract statistically independent components from the EEG signals. For that purpose independent component analysis was applied. Only the most plausible independent components associated with brain activity, with a probability (P) greater than or equal to 50%, were selected and used to reconstruct the data in the electrode space. This reconstruction allowed for subsequent analyses.

The time-series data were segmented into epochs, where each epoch began 1 second before the stimulus onset and ended 2 seconds after the stimulus offset. A frequency-domain analysis is performed, by a Fourier transform of the time data set in the epochs, for each trial.

A primary focus is to compare various coupled conditions within specific frequency bands. Statistical assessments are conducted to determine significant differences among the following conditions:
- Glare vs. low-light condition (in the following it is referred as "Glare effect").
- Glare stimulus with a filter vs. glare stimulus without a filter (in the following it is referred as ("Filter on glare effect").
- Glare stimulus with an optical filter vs. low-light conditions (in the following it is referred as: "Filtered glare on low-light effect").

The frequency bands for the analysis in the statistical study are chosen according to the following ranges:
- Delta band [1 - 4] Hz
- Theta band [4 - 8] Hz
- Alpha band [8 - 12] Hz
- Beta band [12 - 30] Hz
- in the Gamma band: [30 - 49] Hz (i.e. the lower range of the Gamma band).

It must be noted that as the initial statistical study with the multiples bands has shown that the gamma band is the most efficient for the purpose of the assessment of the glare and filters, further statistical study may advantageously be made only with said gamma frequency band.

In the comparison "Filtered glare on low-light effect", the analyses reveal Event-Related Synchronization (ERS) across all examined frequency bands. The spatial activity observed, exhibits a focalized but consistent pattern across these bands. Notably, this pattern is prominently evident in the Fz electrode and the occipital region. Furthermore, in the alpha, beta, and gamma frequency ranges, this activity extended to the central region, particularly focusing on electrode C4.

In the comparison "Filter on glare effect", the analyses reveal Event-Related Desynchronization (ERD) within the delta, theta, and gamma frequency bands. Within the lower-frequency bands, this desynchronization exhibits a focalized pattern primarily in the occipital and frontal regions, specifically at electrodes O2, Fz, and F3. However, in the highest-frequency range, i.e. the gamma band, the desynchronization manifests in a non-focalized but widespread manner, spanning across most cortical regions. Notably, no significant differences were observed between the two conditions of the comparison in the alpha and beta frequency bands.

In the fifth phase of Classification, it is used a machine-learning method (SVM) designed to distinguish between conditions of glare and no glare on a single trial basis. The same machine-learning method (SVM) is also designed, to evaluate the effectiveness of optical filters in alleviating discomfort caused by glare. The classifier is based on EEG data, utilizing the total energy in a specific frequency band, preferably the gamma band, or the full spectrum as features.

Another embodiment can be utilizing single features in all 32 EEG channels with the classifier to achieves near-perfect accuracy in distinguishing between glare and no glare conditions. The gamma frequency band has been found to be the most effective for this classification.

In addition, the predictive power from the Fourier transform signal, is most pronounced in the occipital and prefrontal regions. It has been found that that using approximately five channels in the occipital and frontal, notably prefrontal, regions, the classifier gives an accuracy reaching up to 95%. Using a single channel, the classifier achieves around 75% accuracy. Using all 32 channels, the classifier gives an accuracy approaching 100%. Moreover, it has also been found that the occipital channel 31, when used alone, provides an accuracy of more than 80% with the gamma band.

As regards Region-Specific Efficacy for Filter Evaluation, it has been found that for assessing the effectiveness of optical filters, the prefrontal and frontal regions are the areas where the gamma band gives the highest classification accuracy. The optimal number of channels for this classification is found to be two, simplifying the system while maintaining high effectiveness.

It must be noted that in the current invention, the following implementations are also considered for step c) : determining whether the subject is or not in a no-glare or a discomfort glare state; determining whether the subject is or not in a no-glare or disability glare state; determining whether the subject is or not in a discomfort glare or disability glare state.

## Claims

1. A method for objectively assessing discomfort glare or disability glare of a subject, comprising the steps of:
a) measuring and recording a cortical activity signal of the subject while at least one eye of the subject receives a given light condition during a time comprised between 100 ms and 500 ms,
b) processing the recorded cortical activity signal to obtain a useful signal and to identify, from the useful signal, a specific cortical signature indicative to glare states,
c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state,
**characterized in that** the useful signal which is used to identify the specific cortical signature in step b) comprises a gamma frequency band between 30 Hz and 49 Hz.

2. The method according to claim 1, wherein the useful signal which is used to identify the specific cortical signature in step b) is limited to the gamma frequency band between 30 and 49 Hz to the exclusion of any other frequencies.

3. The method according to anyone of claims 1 and 2, wherein the cortical activity signal which is measured and recorded in step a) comprises regional parts originating from a plurality of different regions of the cortex and wherein the useful signal which is used to identify the specific cortical signature in step b) is obtained from at least these regional parts of the cortical activity signal originating from different regions of the cortex.

4. The method according to claim 3, wherein the cortical activity signal which is measured and recorded in step a) comprises two regional parts originating respectively from the occipital and frontal regions of the cortex and wherein the useful signal which is used to identify the specific cortical signature in step b) is obtained mainly from these two regional parts of the cortical activity signal.

5. The method according to anyone of claims 1 and 2, wherein the cortical activity signal which is measured and recorded in step a) comprises an occipital part originating from an occipital region of the cortex and wherein the useful signal which is used to identify the specific cortical signature in step b) is obtained mainly from this occipital part of the cortical activity signal.

6. The method according to anyone of claims 1, 2, wherein the cortical activity signal which is measured and recorded in step a) comprises a frontal part originating from a frontal region of the cortex and wherein the useful signal which is used to identify the specific cortical signature in step b) is obtained mainly from this frontal part of the cortical activity signal.

7. The method according to anyone of claims 1 to 6, wherein the specific cortical signature is a Fourier transform of the useful signal, said Fourier transform comprising frequency peaks of determined energies, and wherein the predefined threshold parameter is a set of values descriptive of a peak energies repartition over frequencies.

8. The method according to anyone of claims 1 to 7, wherein the threshold parameter of the specific cortical signature is predetermined in a preliminary threshold determination step comprising a statistical computation of data related to cortical activities under different light conditions of a plurality of individuals from a population distinct from the subject whose discomfort or disability glare is to be assessed, in relation to the feeling of each individual as to whether he/she feels a no-glare or discomfort glare or disability glare state.

9. The method according to claim 8, wherein the preliminary threshold determination step comprises the sub-steps of:
-- for each individual of the population, measuring and recording a plurality of cortical activity signals of the individual while at least one eye of the individual receives respective light conditions during a time comprised between 100 ms and 500 ms,
-- for each individual of the population and for each of the light conditions, recording the feeling of the individual as to whether he/she feels a no-glare or discomfort glare or disability glare state,
-- processing the recorded cortical activity signals to obtain useful signals,
-- comparing with each other the useful signals and feelings of each individual or of all the individuals of the population to statistical compute said predefined threshold parameter of the specific cortical signature for which a change occurs between no-glare, discomfort glare or disability glare states in the population of the individuals.

10. The method according to anyone of claims 1 to 9, wherein the threshold parameter of the specific cortical signature is predetermined in a preliminary threshold determination step comprising a statistical computation of data related to cortical activities under different light conditions of the subject whose discomfort or disability glare is to be assessed, in relation to the feeling of the subject as to whether he/she feels a no-glare or discomfort glare or disability glare state.

11. The method according to claim 10, wherein the preliminary threshold determination step comprises the sub-steps of:
-- measuring and recording a plurality of cortical activity signals of said subject while at least one eye of the subject receives respective light conditions during a time comprised between 100 ms and 500 ms,
-- for each of the light conditions, recording the feeling of the subject as to whether he/she feels a no-glare or discomfort glare or disability glare state,
-- processing the recorded cortical activity signals to obtain useful signals,
-- comparing with each other the recorded cortical activity signals and feelings of said subject to determine said predefined threshold parameter of the specific cortical signature as the recorded cortical activity signal for which a change occurs between no-glare, discomfort glare or disability glare states.

12. Method according to anyone of claims 1 to 11, further comprising a step of determining, based on the result the determining at step c), a parameter that is characteristic of a light filter to be provided to the subject in order to maintain or improve the visual comfort and/or visual acuity of the subject.

13. A device for objectively assessing discomfort glare or disability glare of a subject, comprising a control unit configured to execute the steps of:
a) measuring and recording a cortical activity signal of the subject while at least one eye of the subject receives a given light condition during a time comprised between 100 ms and 500 ms,
b) processing the recorded cortical activity signal to obtain a useful signal and to identify, from the useful signal, a specific cortical signature indicative to glare states,
c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state, and
in which the useful signal which is used to identify the specific cortical signature in step b) comprises a gamma frequency band between 30 Hz and 49 Hz.

14. The device according to claims 13, further comprising at least one light source that is operated by the control unit to expose the at least one eye of the subject to said given light condition.

15. An eyewear comprising:
- a device for objectively assessing discomfort glare or disability glare of a subject and comprising a control unit configured to execute the steps of:
a) measuring and recording a cortical activity signal of the subject while at least one eye of the subject receives a given light condition during a time comprised between 100 ms and 500 ms,
b) processing the recorded cortical activity signal to obtain a useful signal and to identify, from the useful signal, a specific cortical signature indicative to glare states,
c) determining whether the subject is or not in a no-glare or a discomfort glare or disability glare state by comparing the specific cortical signature to at least one predefined threshold parameter of the specific cortical signature that is characteristic of a shift from no-glare state to discomfort glare state or disability glare state, and
in which the useful signal which is used to identify the specific cortical signature in step b) comprises a gamma frequency band between 30 Hz and 49 Hz, and
- an active filter having a variable range of transmission,
wherein the control unit is further adapted to determine a value of said variable range of transmission of the active filter based on the result of the assessment by said device of the discomfort glare or disability glare state.
